# EUROPEAN PATENT SPECIFICATION

(11) **EP 4 271 981 B1**
(45) Date of publication and mention of the grant of the patent: **26.08.2026**
(21) Application number: 21915849.0
(22) Date of filing: 29.12.2021
(51) Int. Cl.: G01N 21/64, G01N 21/27, G01J 3/44

(54) **OPTICAL SPECTROMETRY-BASED METHOD AND DEVICE FOR DETECTING TARGET ANALYTE IN SAMPLE**
AUF OPTISCHER SPEKTROMETRIE BASIERENDES VERFAHREN UND VORRICHTUNG ZUM NACHWEIS EINES ZIELANALYTEN IN EINER PROBE
PROCÉDÉ ET DISPOSITIF FONDÉS SUR LA SPECTROMÉTRIE OPTIQUE POUR LA DÉTECTION D'UN ANALYTE CIBLE DANS UN ÉCHANTILLON

(30) Priority: 30.12.2020 KR 20200188382
(43) Date of publication of application: 08.11.2023
(73) Proprietor: Seegene, Inc., Seoul 05548 (KR)
(72) Inventor: KIM, Jae Young, Seoul 07703 (KR)
(74) Representative: Santarelli
(86) International application number: PCT/KR2021/020218
(87) International publication number: WO 2022/146052

(56) References cited:
- KR-A- 20080 100 343
- KR-A- 20110 040 743
- US-A1- 2003 138 875
- US-A1- 2007 161 876
- US-A1- 2013 122 607
- US-A1- 2013 169 968
- US-A1- 2017 082 421
- US-B1- 9 435 687
- US-B2- 10 194 854
- HOFSTRAAT J W ET AL: "CORRECTION OF FLUORESCENCE SPECTRA", APPLIED SPECTROSCOPY, THE SOCIETY FOR APPLIED SPECTROSCOPY. BALTIMORE, US, vol. 48, no. 4, April 1994 (1994-04-01), pages 436 - 447, XP000439472, ISSN: 0003-7028

## Description

### [Technical Field]

The present disclosure relates to an optical spectrometry-based method and device for detecting a target analyte in a sample.

### [Background Art]

In modern times, with increasing interest in health, as well as increasing life expectancy, the importance of and demand for nucleic acid-based in vitro molecular diagnostics, such as accurate analysis of pathogens and genetic analyses of patients, are increasing.

Nucleic acid-based molecular diagnostics is carried out by extracting nucleic acids from a sample and identifying the presence of a target nucleic acid within the extracted nucleic acids. A sample processing process of extracting nucleic acids from a sample includes sequentially mixing the sample with a variety of reagents and removing residues other than the nucleic acids. Nucleic acid amplification reaction, which is well-known as polymerase chain reaction (PCR), includes a repeated cycle process comprised of denaturation of a doublestranded deoxyribonucleic acid (DNA), annealing of an oligonucleotide primer to a DNA template, and primer extension by DNA polymerase (Mullis et al., U.S. Patent Nos. 4,683,195, 4,683,202, and 4,800,159; and Saiki et al., 1985, Science 230, PP. 1350-1354).

A fluorescent material which is an optical label included in samples emits fluorescence acting as an optical marker. A light source unit emits excitation light to the samples, and the fluorescent material excited by the excitation light emits the fluorescence. In order to emit excitation light having a specific wavelength to the samples, a filter may be disposed on an optical path of the excitation light. In addition, in order to accurately detect the fluorescence emitted by the fluorescent material exited by the excitation light, a filter may also be disposed on an optical path of the emission light.

Respective optical labels react to excitation light within a unique wavelength range, and emit emission light in a unique wavelength range. Thus, a variety of expensive filters should be used in order to detect a variety of fluorescent materials.

In addition, in an optical spectroscopy instrument irradiating a sample with excitation light and detecting emission light generated from the sample, excitation light should also be detected when emission light is detected by a detection unit, due to the optical path structure. In order to prevent this, the detection unit uses a filter allowing light having an emission light wavelength to pass therethrough. However, since a portion of the spectrum of the excitation light of the fluorescent material overlaps a portion of the spectrum of the emission light, it is impossible to completely detect emission light by separating the emission light from the excitation light, even using a filter. In addition, when only the wavelength unique to the emission light is filtered, a significant quantity of the emission light may be lost.

Therefore, there is demand for the development of a novel device or method able to effectively measure the quantity of emission light generated by an optical label due to the presence of a target analyte in a sample from light measured by a detection unit.

Document US 9435687 B1 discloses a method to remove the spectral components of illumination energy from a sample spectrum without the use of optical barrier filters, and an apparatus for the same.

Document US 2007/161876 A1 discloses a method and an apparatus for rapid detection and diagnosis of tissue abnormalities.

Document US 10194854 B2 discloses a method for non-invasive monitoring of fluorescent tracer agent with background separation corrections.

Document US 2003/138875 A1 discloses a method and apparatus for detecting the presence of microbes and determining their physiological status.

### [Disclosure]

### [Technical Problem]

In consideration of the above-described issues, the present inventors have developed a method and device able to measure emission light by selectively separating the emission light from light detected by a detection unit. As a result of performing optical spectrometry by changing the quantity of light generated by a light source unit, the present inventors have discovered that, when the overall quantity of light is changed, there is a change in the quantity of light of each wavelength, but the ratio of the quantity of light between respective wavelengths is maintained without a significant change. In addition, it has also been discovered that this regularity appears in light emitted by optical labels. Based on this feature, the present inventors have developed a method of detecting a target analyte in a sample by measuring quantities of light for a plurality of different preset wavelengths previously determined for excitation light generated by the light source unit and measured light detected through the sample and determining emission light generated by an optical label in the sample using the measured quantities of light. The present inventors have also developed a device for detecting a target analyte in a sample using spectrometry, the device including an optical spectrometer unit to perform the optical spectrometry.

Accordingly, an objective of the present disclosure is to provide a method of detecting a target analyte in a sample, the method including the following steps of: (a) irradiating a sample with light by generating light by a light source unit of a device for detecting a target analyte, wherein the device for detecting a target analyte includes the light source unit, an optical spectrometer unit, and a sample receiving unit; (b) obtaining a reference light profile and a measured light profile, wherein the reference light profile includes information on the light quantity of the light generated by the light source unit for each preset wavelength of a set of preset wavelengths, and the measured light profile includes information on the light quantity of the light measured from the sample for each preset wavelength of the set of preset wavelengths; (c) determining a mathematical relationship between a light quantity for a reference preset wavelength of the reference light profile and a light quantity for the reference preset wavelength of the measured light profile, wherein the reference preset wavelength is a wavelength not including a wavelength of emission light; (d) determining a contribution extent of an excitation light profile in the measured light profile from the reference light profile and the mathematical relationship determined; (e) obtaining an emission light profile from (i) the contribution extent determined and (ii) the measured light profile; and (f) detecting a target analyte in the sample from the emission light profile.

The above and other objectives and advantages of the present disclosure will be more clearly understood from the following embodiments, claims, and drawings.

### [Technical Solution]

In order to realize at least one of the above-described objectives, the present disclosure provides a method of detecting a target analyte in a sample, the method including: (a) irradiating a sample with light by generating light by a light source unit of a device for detecting a target analyte, wherein the device for detecting a target analyte includes the light source unit, an optical spectrometer unit, and a sample receiving unit; (b) obtaining a reference light profile and a measured light profile, wherein the reference light profile includes information on the light quantity of the light generated by the light source unit for each preset wavelength of a set of preset wavelengths, and the measured light profile includes information on the light quantity of the light measured from the sample for each preset wavelength of the set of preset wavelengths; (c) determining a mathematical relationship between a light quantity for a reference preset wavelength of the reference light profile and light quantity for the reference preset wavelength of the measured light profile, wherein the reference preset wavelength is a wavelength not including a wavelength of emission light; (d) determining a contribution extent of an excitation light profile in the measured light profile from the reference light profile and the mathematical relationship determined; (e) obtaining an emission light profile from (i) the contribution extent determined and (ii) the measured light profile; and (f) detecting a target analyte in the sample from the emission light profile.

The invention also concerns a computer readable storage medium according to claim 7, a device according to claim 8 and a computer program to be stored on a computer readable storage medium according to claim 9.

### [Advantageous Effects]

According to the present disclosure, it is possible to accurately detect a target nucleic acid by measuring emission light by effectively separating the emission light from measured light which is measured by the detection unit, and from which excitation light is removed.

According to the present disclosure, it is not necessary to essentially use an optical filter for filtering a specific wave of excitation light or emission light.

### [Description of Drawings]

FIG. 1 is a diagram illustrating the configuration of a device for detecting a target analyte according to an embodiment of the present disclosure;
FIG. 2 illustrates signals according to preset wavelengths detectable by the optical spectrometer unit according to an embodiment of the present disclosure;
FIG. 3 compares the distribution of wavelengths of extication light generated by a light source unit, the distribution of wavelengths of measured light, and the distribution of wavelengths of light emitted by an optical label in an example of the present disclosure; and
FIGS. 4A to 4D illustrate a reference light profile, an excitation light profile, the contribution extent of the excitation light profile, and an emission light profile.

### [Mode for Invention]

Hereinafter, embodiments of the present disclosure will be described in more detail. The embodiments set forth herein are only provided for illustrative purposes to fully convey the concept of the present disclosure, and it would be obvious to those having ordinary knowledge in the art that the scope of the present disclosure is not limited to these embodiments, according to the concept of the present disclosure.

In designating elements of the drawings by reference numerals, the same elements will be designated by the same reference numerals although they are shown in different drawings. Further, in the following description of the present disclosure, a detailed description of known functions and configurations incorporated herein will be omitted in the case in which the subject matter of the present disclosure may be rendered unclear thereby.

Terms, such as "first", "second", "A", "B", "(A)", or "(B)" may be used herein to describe elements of the present disclosure. Each of these terms is not used to define the essence, order, sequence, or number of elements etc., but is used merely to distinguish the corresponding element from other elements. In the case in which it is described that a first element is "connected", "coupled", or "joined" to a second element, not only can the first element be directly "connected", "coupled", or "joined" to the second element, but a third element can also be "interposed" between the first and second elements.

### I. Optical Spectrometry-Based Method of Analyzing Target in Sample

According to an aspect of the present disclosure, provided is a method of detecting a target analyte in a sample, the method including the following steps of: (a) irradiating a sample with light by generating light by a light source unit of a device for detecting a target analyte, wherein the device for detecting a target analyte includes the light source unit, an optical spectrometer unit, and a sample receiving unit; (b) obtaining a reference light profile and a measured light profile, wherein the reference light profile includes information on the light quantity of the light generated by the light source unit for each preset wavelength of a set of preset wavelengths, and the measured light profile includes information on the light quantity of the light measured from the sample for each preset wavelength of the set of preset wavelengths;
(c) determining a mathematical relationship between a light quantity for a reference preset wavelength of the reference light profile and a light quantity for the reference preset wavelength of the measured light profile, wherein the reference preset wavelength is a wavelength not including a wavelength of emission light; (d) determining a contribution extent of an excitation light profile in the measured light profile from the reference light profile and the mathematical relationship determined; (e) obtaining an emission light profile from (i) the contribution extent determined and (ii) the measured light profile; and (f) detecting a target analyte in the sample from the emission light profile.

The present disclosure relates to the method of detecting a target analyte in a sample.

The sample includes biological samples (e.g., cells, tissue, and fluid from a biological source) and non-biological samples (e.g., food, water, and soil). The biological samples include, not limited to, viruses, bacteria, tissue, cells, blood, serum, plasma, lymph, sputum, swab, aspirate, bronchioalveolar lavage fluid, milk, urine, feces, ocular fluid, saliva, semen, brain extracts, spinal cord fluid (SCF), extracts from the appendix, spleen, and tonsillar tissue, amniotic fluid, and ascitic fluid. In addition, the sample may include naturally-occurring nucleic acid molecules isolated from biological sources and synthetic nucleic acid molecules. According to an embodiment of the present disclosure, the sample may include an additional substance such as water, deionized water, saline water, a pH buffer solution, an acid solution, or a base solution.

The target analyte according to the present disclosure refers to a material including a variety of materials (e.g., biological materials and non-biological materials), particularly, biological materials, and more particularly, materials including nucleic acid molecules (e.g., deoxyribonucleic acid (DNA) and ribonucleic acid (RNA)), proteins, peptides, carbohydrates, lipids, amino acids, biological compounds, hormones, antibodies, antigens, metabolites, and cells. The target analyte includes materials existing in nature or synthetic materials. Most particularly, the target analyte may be target nucleic acid molecules. The target analyte may be contained in the sample.

The detection of the target analyte in the sample refers to determining the presence or absence of the target analyte in the sample. The determination of the presence or absence of the target analyte in the sample may be qualitative determination or quantitative determination. The determination of the presence or absence of the target analyte in the sample may include performing amplification reaction, for example, PCR, real-time PCR, or isothermal amplification reaction (e.g., LAMP or RPA) on the sample in which the presence or absence of the target analyte is to be confirmed and then determining the presence or absence of the target analyte in the sample from the result of the amplification reaction.

The amplification reaction for amplifying signals indicating the presence of the target analyte, particularly, target nucleic acid molecules may be performed in such a manner that signals are amplified simultaneously with the amplification of the target analyte (e.g., real-time PCR). Alternatively, according to an example, the amplification reaction may be performed in such a manner that the target analyte is not amplified but signals indicating the presence of the target analyte are only amplified (e.g., cycling probe technologies (CPT) (Duck P, et al., Biotechniques, 9:142-148(1990)), Invader Assay (U.S. Patent Nos. 6,358,691 and 6,194,149).

In addition, the target analyte, particularly, target nucleic acid molecules may be amplified by a variety of methods including, but not limited to, polymerase chain reaction (PCR), ligase chain reaction (LCR) (U.S Patent Nos. 4,683,195 and 4,683,202, PCR Protocols: A Guide to Methods and Applications (Innis et al., eds, 1990)), strand displacement amplification (SDA) (Walker, et al. Nucleic Acids Res. 20(7):1691-6 (1992)); Walker PCR Methods Appl. 3(1):1-6 (1993)) transcription-mediated amplification (Phyffer, et al., J. Clin. Microbiol. 34:834-841 (1996); Vuorinen, et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatchet al., Genet. Anal. 15(2):35-40 (1999)), and Q-Beta Replicase (Lizardi et al., BiolTechnology 6:1197 (1988)).

The above-described result refers to the presence/absence or intensity of a signal changing depending on the presence or absence of the target analyte or the quantity of the target analyte in the sample. In the present disclosure, the signal is an optical signal. The optical signal may be a signal generated by the optical label. For example, the optical label may be one or more optical labels selected from the group consisting of, but is not limited to, FAM^{™}, TET^{™}, VIC^{™}, JOE^{™}, HEX^{™}, CY3, TAMRA^{™}, ROX^{™}, Texas Red, CY5, CY5.5, and Quasar 705.

A variety of methods of generating an optical signal indicating the presence of target nucleic acid molecules using nucleic acid reaction is known. Representative examples may include, but are not limited to, the TaqMan^{™} probe method (U.S. Patent No. 5,210,015), the molecular beacon method (Tyagi et al., Nature Biotechnology v.14 March 1996), the Scorpion method (Whitcombe et al., Nature Biotechnology 17:804-807 (1999)), the Sunrise or Amplifluor method (Nazarenko et al., 2516-2521 Nucleic Acids Research, 25(12):2516-2521 (1997) and U.S. Patent No. 6,117,635), the Lux method (U.S. Patent No. 7,537,886), the CPT method (Duck P, et al., Biotechniques, 9:142-148 (1990)), the LNA method (U.S. Patent No. 6,977,295), the Plexor method (Sherrill CB et al., Journal of the American Chemical Society, 126:4550-4556 (2004)), Hybeacons^{™} (D. J. French et al., Molecular and Cellular Probes (2001) 13, 363-374 and U.S. Patent No. 7,348,141), the dual-lebeled, self-quenched probe (U.S. Patent No. 5,876,930), the hybridization probe (Bernard PS, et al., Clin Chem 2000, 46, 147-148), the PTO cleavage and extension (PTOCE) method (WO2012/096523), the PTO cleavage and extension-dependent signaling oligonucleotide hybridization (PCE-SH) method (WO2013/115442), the PTO cleavage and extension-dependent non-hybridization (PCE-NH) method (PCT/KR2013/012312), and the CER method (WO2011/037306).

Hereinafter, the method according to the present invention will be described in a step by step manner.
(a) irradiating a sample with light by generating light by a light source unit of a device for detecting a target analyte

The device for detecting a target analyte is a device for detecting an optical signal from the sample. Specifically, the device for detecting a target analyte may be a device configured to generate an optical signal depending on the presence of the target analyte in the sample by irradiating the sample with light and detect the optical signal generated.

According to an embodiment of the present disclosure, the optical signal may be an optical signal indicating the presence of the target analyte in the sample, in particular, the presence of a target nucleic acid. Thus, the device according to the present disclosure may be a target nucleic acid detection device.

The device for detecting a target analyte includes a light source unit, an optical spectrometer unit, and a sample receiving unit.

The light source unit provides light energy required for the detection. The light source unit includes a light source. The light source may be a plurality of light source units. The plurality of light source may be configured to generate light of different wavelengths. In this case, power may be selectively applied to the light unit, so that the light source unit may provide light of an intended excitation wavelength.

The optical spectrometer unit includes a spectrometer configured to transmit a signal regarding the quantity of light for a preset wavelength with respect to incident light. The optical spectrometer unit measures the quantity of light for each preset wavelength of a set of preset wavelengths.

The sample receiving unit receives the sample itself or a container containing the sample.

According to an embodiment of the present disclosure, the method according to the present disclosure may include a step of positioning the sample in the sample receiving unit of the device for detecting a target analyte before the step (a). Positioning the sample in the sample receiving unit may be a step of positioning the container containing the sample in the sample receiving unit. The container may be, for example, a test tube, a PCR tube, a tube including a strip tube, a vial, a multi-well plate, a microplate, a slide glass, a microarray, or a cartridge.

The sample or the container containing the sample is positioned in the sample receiving unit, and then the sample is irradiated with light by generating the light by the light source unit.

Irradiating the sample with light may be an operation of irradiating onto the sample receiving unit with light from the light source unit. The light is generated by the light source unit and irradiate the sample or the sample receiving unit through at least one of a filter or a lens. The light generated by the light source unit may directly irradiate the sample or irradiate the sample receiving unit. In addition, the light may irradiate the sample or the sample receiving unit, for example, by a beam splitter, a mirror, an optic cable, or an optic fiber.

The device for detecting a target analyte and components thereof will be described in detail in Section II below.

The light generated by the light source unit is excitation light. The excitation light generates emission light depending on the presence of the target analyte in the sample.

According to an embodiment of the present disclosure, the sample may include an optical label.

The optical label refers to a label that generates an optical signal depending on the presence of a target nucleic acid. The optical label may be a fluorescence label. The fluorescence label usable in the present disclosure may include any molecules known in the art. Examples of the fluorescence label may include, but are not limited to, Cy2^{™} (506), YO-PRO^{™}-1 (509), YOYO^{™}-1 (509), Calcein (517), FITC (518), FluorX^{™} (519), Alexa^{™} (520), Rhodamine 110 (520), Oregon Green^{™} 500 (522), Oregon Green- 488 (524), RiboGreen^{™} (525), Rhodamine Green^{™} (527), Rhodamine 123 (529), Magnesium Green^{™} (531), Calcium Green^{™} (533), TO-PRO^{™}-1 (533), TOTO1 (533), JOE (548), BODIPY530/550 (550), Dil (565), BODIPY TMR (568), BODIPY558/568 (568), BODIPY564/570 (570), Cy3^{™} (570), Alexa^{™} 546 (570), TRITC (572), Magnesium Orange^{™} (575), Phycoerythrin R&B (575), Rhodamine Phalloidin (575), Calcium Orange^{™}(576), Pyronin Y (580), Rhodamine B (580), TAMRA (582), Rhodamine Red^{™} (590), Cy3.5^{™} (596), ROX (608), Calcium Crimson^{™} (615), Alexa^{™} 594 (615), Texas Red(615), Nile Red (628), YO-PRO^{™}-3 (631), YOYO^{™}-3 (631), R-phycocyanin (642), C-Phycocyanin (648), TO-PRO^{™}-3 (660), TOTO3 (660), DiD DilC(5) (665), Cy5^{™} (670), Thiadicarbocyanine (671), Cy5.5 (694), HEX (556), TET (536), Biosearch Blue (447), CAL Fluor Gold 540 (544), CAL Fluor Orange 560 (559), CAL Fluor Red 590 (591), CAL Fluor Red 610 (610), CAL Fluor Red 635 (637), FAM (520), Fluorescein (520), Fluorescein-C3 (520), Pulsar 650 (566), Quasar 570 (667), Quasar 670 (705), and Quasar 705 (610). Each numeric in parentheses is a maximum emission wavelength expressed in nanometers.

The optical label includes both a single label system and an interactive label system. The interactive label system is a signal generating system in which energy is transferred non-radioactively between a donor molecule (i.e., a reporter molecule) and an acceptor molecule (i.e., a quencher molecule). In addition, the reporter molecule may be fluorescent, whereas the quencher molecule may be non-fluorescent. For example, a non-fluorescent dark quencher capable of quenching fluorescence of a wide wavelength or a specific wavelength may be used in the present disclosure. When the quencher molecule is fluorescent, a target nucleic acid sequence may be detected from a signal change of the fluorescent quencher molecule.

The optical label according to the present disclosure is excited by excitation light of a specific wavelength, and thus generates emission light of the specific wavelength. The method according to the present invention includes a step of measuring, by the optical spectrometer unit, the light generated by the light source unit. The light generated by the light source unit does not include emission light emitted from the sample. A reference light profile may be obtained by measuring, by the optical spectrometer unit, the light generated by the light source unit.

The method according to the present invention includes a step of measuring, by the optical spectrometer unit, light from the sample. The light measured from the sample may be light including both excitation light that has reached the sample from the light source unit and emission light generated from the sample. A measured light profile is obtained by measuring the light from the sample by the optical spectrometer unit.

### (b) obtaining a reference light profile and a measured light profile

The reference light profile includes information on the light quantity of the light generated by the light source unit for each preset wavelength of a set of preset wavelengths, whereas the measured light profile includes information on the light quantity of the light measured from the sample for each preset wavelength of the set of preset wavelengths.

The method according to the present invention obtains optical information necessary for the detection for each preset wavelength.

An optical signal measured from the sample includes not only an optical signal (i.e., emission light) generated depending on the presence or amount of the target analyte in the sample, but also excitation light generated by the light source unit to irradiate the sample in order to excite the optical label. In the method according to the present invention, in order to measure the emission light by separating the emission light from the optical signal measured from the sample, the present method obtains the optical signal generated from the sample by obtaining information on the light quantity for each of wavelengths separated by bands, determining the contribution extent of the excitation light included in the measured light using the obtained information, and removing the contribution extent of the excitation light from the measured light.

The preset wavelength means a predetermined wavelength. The preset wavelength may be, for example, a range of visible light (e.g., from about 370 nm to about 780 nm) or any suitable wavelength belonging to a range based on the range of visible light and a range of infrared radiation (e.g., from about 780 to about 1300 nm). The wavelength bandwidth of the preset wavelength may be equal to or greater than 5 nm, 10 nm, 20 nm, or 30 nm and equal to or smaller than 500 nm, 400 nm, 300 nm, 200 nm, or 100 nm, although the wavelength bandwidth is not specifically limited.

A set of preset wavelengths refers to a set of a plurality of preset wavelengths. The set of preset wavelengths includes a plurality of different preset wavelengths. The preset wavelengths of the set of preset wavelengths may partially overlap each other. For example, when a first preset wavelength ranges from 400 nm to 460 nm, a second preset wavelength may range from 430 nm to 480 nm. At least two present wavelengths of the set of preset wavelengths may have different wavelength bandwidths. For example, the wavelength bandwidth of the first preset wavelength may be 60 nm, whereas the wavelength bandwidth of the second preset wavelength may be 50 nm.

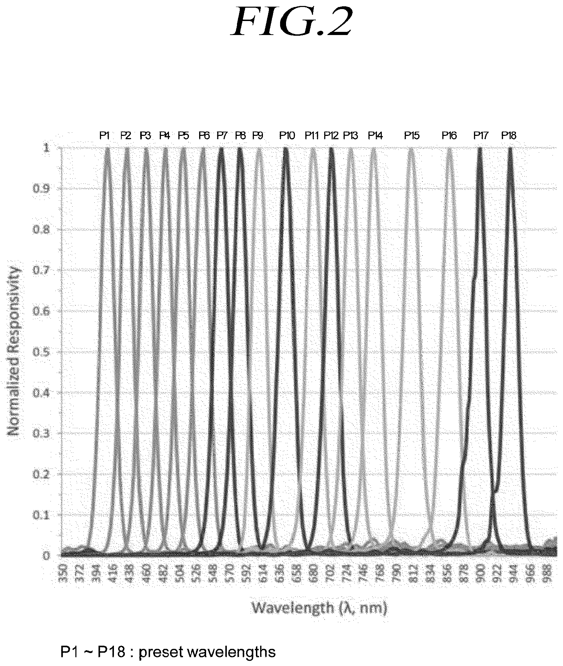

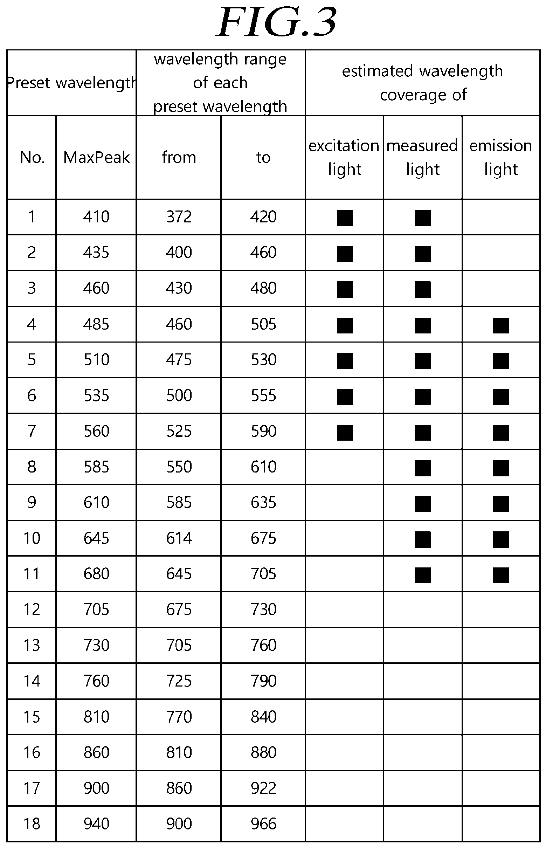

A wavelength covered by the set of preset wavelengths may be a range including the entirety or a portion of the wavelength of excitation light necessary to excite the optical label used for detecting the target analyte. The wavelength covered by the set of preset wavelengths may be a range including the entirety or a portion of the wavelength of emission light generated by the optical label. For example, the optical label may be one or more optical labels selected from the group consisting of, but is not limited to, FAM^{™}, TET^{™}, VIC^{™}, JOE^{™}, HEX^{™}, CY3, TAMRA^{™}, ROX^{™}, Texas Red, CY5, CY5.5, and Quasar 705. FIG. 3 illustrates settings of preset wavelengths according to an example. In FIG. 3, 18 preset wavelengths are set in order to cover a wavelength range of from about 370 nm to about 970 nm. FIG. 2 illustrates signals according to the preset wavelengths obtained by the optical spectrometer unit according to an embodiment of the present disclosure.

According to an embodiment of the present disclosure, a plurality of preset wavelength of the set of preset wavelengths may cover the entirety of a wavelength range overlapping the wavelength of the emission light emitted by the optical label among the wavelength of light that has reached the sample from the light source unit, and at least one preset wavelength may be set to be included in a wavelength range not overlapping the wavelength of emission light emitted by the optical label among the wavelength of light that has reached the sample from the light source unit. For example, in the case of the detection of the target analyte in which the optical label (e.g., FAM) emitting emission light of a wavelength ranging from about 470 nm to about 670 nm is used and the light source unit generates light of a wavelength ranging from about 400 nm to about 540 nm to excite the optical label, the set of preset wavelengths includes a plurality of preset wavelengths to cover the entirety of a wavelength ranging from 470 nm to 540 nm among the wavelength of light generated by the light source unit, overlapping the wavelength of the emission light. In addition, the set of preset wavelengths may be set to include at least one preset wavelength included in a wavelength ranging from 400 nm to 470 nm among the wavelength of the light generated by the light source unit, not overlapping the wavelength of the emission light. For example, the set of preset wavelengths may include preset wavelengths which are previously set to 400 nm to 460 nm, 430 nm to 480 nm, 460 nm to 505 nm, 475 nm to 530 nm, and 500 nm to 555 nm, respectively.

According to an embodiment of the present disclosure, the plurality of preset wavelength of the set of preset wavelengths may be set to cover the entirety of the wavelength of light reaching the sample from the light source unit. For example, in the case of the detection of the target analyte in which the light source unit generates light of a wavelength ranging from about 400 nm to about 540 nm, the set of preset wavelengths may include a plurality of preset wavelengths to cover the entirety of the wavelength of from about 400 nm to about 540 nm.

According to an embodiment of the present disclosure, the plurality of preset wavelength of the set of preset wavelengths may be set to cover all of the wavelength of light reaching the sample from the light source unit and the wavelength of the emission light emitted by the optical label. For example, in the case of the detection of the target analyte in which the optical label (e.g., FAM) emitting emission light of a wavelength ranging from about 470 nm to about 670 nm and the light source unit generates light of a wavelength ranging from about 400 nm to about 540 nm to excite the optical label, the set of preset wavelengths may include a plurality of preset wavelengths to cover the entirety of the wavelength of from about 400 nm to about 670 nm.

The number of the preset wavelengths of the set of preset wavelengths is not specifically limited, but may be, for example, equal to or greater than 2, 3, 4, 5, 6, 7, 8, 9, or 10 or equal to or smaller than 100, 90, 80, 70, 60, 50, or 40.

According to an embodiment of the present disclosure, the reference light profile or the measured light profile may include information on a light quantity for additional wavelengths, in addition to the information on a light quantity for each preset wavelength of the set of preset wavelengths.

In the present disclosure, a profile refers to a group of values featuring specific light. Specifically, the profile according to the present disclosure is a set of information on light quantities of specific light for a plurality of wavelengths. For example, the profile may be a set of information obtained by measuring the intensity of the specific light according to a variety of wavelengths. For example, the measurement may be performed by the optical spectrometer unit, such as an optical sensor, capable of separately detecting light of a variety of wavelengths. Alternatively, the measurement may be performed using a plurality of filters each selectively allowing light of each preset wavelength to pass therethrough or a detection module including a plurality of optical sensors each selectively detecting light of each preset wavelength.

The information on light quantities may be information obtained by measuring optical properties, such as luminous flux, luminous intensity, luminance, and illuminance, or may be a conversion value of an electrical signal, such as relative fluorescence unit (RFU) or normalized responsivity (NR), output for light of a corresponding wavelength by a measurement instrument, such as a multi-channel photo-sensor, a spectrometer, or a photodiode.

The reference light profile is a profile for light (i.e., excitation light) generated by the light source unit to excite the optical label included in the sample. The reference light profile is a profile for light that does not include light (i.e., emission light) generated by the optical label in the sample. The reference light profile includes information on a light quantity for each preset wavelength of the set of preset wavelengths for the light generated by the light source unit. The reference light profile is obtained by measuring the light quantity of the light generated by the light source unit for each preset wavelength. The light source unit includes a light source. The light source unit may include a filter filtering light generated by the light source. The light source unit may include a lens configured to allow the light generated by the light source unit to pass therethrough. The measurement of the reference light profile may be performed, for example, by a method of measuring light quantities of light, transferred from the light source unit to the optical spectrometer unit, for respective preset wavelengths using the optical spectrometer unit.

The measured light profile is a profile for light measured from the sample. The light measured from the sample includes both light (i.e., emission light) generated by the optical label in the sample and light (i.e., excitation light) generated by the light source unit to irradiate the sample to excite the optical label. The measured light profile may be obtained, for example, by a method of measuring light transferred from the sample holder to the optical spectrometer unit, particularly, the light quantity of light for each preset wavelength using the optical spectrometer unit. The measured light profile includes information on the light quantity of light generated from the sample for each preset wavelength of the set of preset wavelengths.

According to an embodiment of the present disclosure, the reference light profile and the measured light profile may be obtained using different optical spectrometer units. When the reference light profile and the measured light profile are obtained using different optical spectrometer units, the two profiles may be simultaneously obtained using the identical light source(s).

According to an embodiment of the present disclosure, the reference light profile and the measured light profile may include information on the light quantity for each preset wavelength of the same set of preset wavelengths. For example, when the reference light profile includes information on light quantities for the first preset wavelength ranging from 372 nm to 420 nm and the second preset wavelength ranging from 460 nm to 505 nm, the measured light profile may also include information on light quantities for the same preset wavelengths, i.e., the first preset wavelength ranging from 372 nm to 420 nm and the second preset wavelength ranging from 460 nm to 505 nm.

Information on the light quantity for the same preset wavelength may be measured from reference light and measured light, and the contribution extent of excitation light for each preset wavelength included in the measured light may be calculated by comparing results.

According to an embodiment of the present disclosure, the reference light profile and the measured light profile may be obtained using the same light source unit(s). The reference light profile is obtained by measuring light transferred to the detection module, such as the optical spectrometer unit, from the light source unit, whereas the measured light profile is obtained by measuring light transferred to the optical spectrometer unit from the sample receiving unit irradiated with light from the light source unit.

According to an embodiment of the present disclosure, the reference light profile and the measured light profile may be simultaneously obtained using the same light source unit(s). The term "simultaneously obtaining" refers to, when light is generated using power applied to the light source unit, measuring the light quantity of light from light source and from sample for each preset wavelength to obtain the reference light profile and the measured light profile using the light generated. When the reference light profile and the measured light profile are simultaneously obtained using the same light source unit(s), a more accurate measurement is possible.

Since the excitation light measured when obtaining the reference light profile and the excitation light included in the measured light have different measurement positions, the two excitation lights may have different light intensities. However, since the two excitation lights are generated by the same light source unit, the two excitation lights have the same pattern of optical spectrum even if the light intensities are different. For example, when the ratio of the light quantity between the first preset wavelength and the second preset wavelength of the reference light profile is 1:3, the ratio of the concentration extent of the excitation light between the first preset wavelength and the second preset wavelength in the measured light profile is 1:3.

According to an embodiment of the present disclosure, the reference light profile is a set of information on light quantities of light generated by the light source unit for each preset wavelength of a set of preset wavelengths. According to an embodiment of the present disclosure, the measured light profile is a set of information on light quantities of light measured from the sample for each preset wavelength of a set of preset wavelengths. The reference light profile and the measured light profile may include the same set of preset wavelengths. According to an embodiment of the present disclosure, the measured light profile or the reference light profile may include other preset wavelengths in addition to the set of preset wavelengths.

According to an embodiment of the present disclosure, obtaining the reference light profile and the measured light profile may be obtaining a plurality of reference light profiles and a plurality of measured light profiles in different measurement conditions. The measurement conditions may include, for example, the light irradiation time of the light source unit, the sensitivity of the optical spectrometer unit, the intensity of an electrical signal output in response to detection of light, and the like. The intensity of emission light generated by either the light source or the sample may exceed a range that the sensor can detect, and a plurality of measurements according to a variety of measurement conditions may correct such an error. A final reference light profile and a final measured light profile may be obtained by combining the plurality of profiles obtained in these different measurement conditions. According to an embodiment of the present disclosure, obtaining the reference light profile and the measured light profile may be obtaining the plurality of reference light profiles and the plurality of measured light profiles in which the light source unit has different light irradiation times. According to an embodiment of the present disclosure, obtaining the reference light profile and the measured light profile may be obtaining the plurality of reference light profiles and the plurality of measured light profiles by changing the gains of the optical spectrometer unit.

(c) determining a mathematical relationship between a light quantity for a reference preset wavelength of the reference light profile and a light quantity for the reference preset wavelength of the measured light profile

In the step (c), the mathematical relationship is determined from the light quantity for the reference preset wavelength of the reference light profile and the light quantity for the reference preset wavelength of the measured light profile.

The reference preset wavelength is one or more preset wavelength selected from the set of preset wavelengths. The reference preset wavelength is determined from preset wavelengths each of which does not include the wavelength of the emission light. The reference preset wavelength may be a plurality of reference preset wavelengths. The reference preset wavelength may be a preset wavelength not including the wavelength of the emission light. In the preset wavelength of the measured light profile not including the wavelength of the emission light, light reaching the sample from the light source unit is measured, and the emission light generated from the sample is not measured. Thus, the entirety of the light quantities measured in the reference preset wavelength is light generated by the light source unit.

Thus, the relationship between the light quantity for the reference preset wavelength of the reference light profile and the light quantity for the reference preset wavelength of the measured light profile indicates the relationship between excitation light generated by the light source unit included in the measured light and reference light. Thus, the contribution extent of the excitation light in the measured light may be calculated using the relationship determined.

An example of determining the reference preset wavelength will be described with reference to FIG. 3. Referring to FIG. 3, 18 preset wavelengths are set as an example. The wavelength of the light source may be determined by the user in consideration of the optical label to be used. In FIG. 3, a light source generating light of first to seventh preset wavelengths is used. In addition, the wavelength of the emission light is determined depending on the optical label to be used by the user. The optical label used in the example of FIG. 3 emits light of fourth to eleventh preset wavelengths. A reference preset wavelength may be determined in accordance with this information. In the example of FIG. 3, one or more preset wavelengths of the first to third preset wavelengths may be determined to be the reference preset wavelength.

According to the invention, the relationship is a mathematical relationship. The relationship may be expressed by a variety of methods. For example, the relationship may be mathematically defined. The relationship may be, for example, a ratio of the intensity between reference light and measured light in a specific wavelength. The relationship may be, for example, the difference in the intensity between the reference light and the measured light in the specific wavelength. The specific wavelength may be a reference preset wavelength. The relationship may be determined from a single light quantity of the reference preset wavelength of the reference light profile and a single light quantity of the reference preset wavelength of the measured light profile, or determined by obtaining a plurality of light quantities for the reference preset wavelength of the reference light profile and the measured light profile. For example, the relationship may be determined from a mean value, a median value, a minimum value, or a maximum value of a plurality of light quantities for the reference preset wavelength of the reference light profile and a mean value, a median value, a minimum value, or a maximum value of a plurality of light quantities for the reference preset wavelength of the measured light profile. When the plurality of values are used in the determination of the relationship, the effect of one abnormal value on the detection may be minimized.

The relationship between the reference light and the excitation light included in the measured light may be a relationship constant irrespective of the preset wavelength to be compared therewith. Alternatively, the relationship may regularly change according to the preset wavelength. Thus, the relationship may be expressed as a function for the preset wavelength. According to an embodiment of the present disclosure, the relationship may be a mathematical function, in which the relationship is expressed as a function of the preset wavelength.

(d) determining a contribution extent of an excitation light profile in the measured light profile from the reference light profile and the determined mathematical relationship.

The contribution extent of the excitation light profile in the measured light profile refers to the degree of the excitation light included in the measured light. The measured light profile includes information on the light quantity for each of a plurality of preset wavelengths. Specifically, the contribution extent of the excitation light profile in the measured light profile refers to the light quantity for a corresponding preset wavelength for the excitation light included in the information on the light quantity for each preset wavelength of the measured light profile.

The measured light profile is measured by detecting not only the emission light generated from the sample but also the excitation light generated by the light source unit. Thus, the contribution extent of the excitation light profile in the measured light profile is first calculated in order to obtain the profile for the emission light.

The present invention provides the contribution extent of the excitation light profile in the measured light profile from the reference light profile and the determined mathematical relationship.

The reference light profile and the contribution extent of the excitation light profile are obtained by measuring light from the same light source unit(s) for each preset wavelength. However, the reference light profile and the contribution extent of the excitation light profile do not have the same light quantity for the same preset wavelength, since the two profiles are obtained on different optical paths. However, since the reference light profile and the excitation light profile are obtained by measuring light from the same light source unit(s) for each preset wavelength, the pattern of light quantities for respective preset wavelengths of the reference light profile is the same as the pattern of light quantities for respective preset wavelengths of the contribution extent of the excitation light profile.

The excitation light profile included in the measured light profile may be provided using the reference light profile and the determined mathematical relationship.

According to an embodiment of the present disclosure, providing the contribution extent of the excitation light profile in the measured light profile may be provided by a method of providing the contribution extent of the excitation light in the corresponding preset wavelength of the measured light profile by applying a function value, calculated by substituting each of the preset wavelengths to the mathematical relationship determined, to information on the light quantity for the corresponding preset wavelength of the reference light profile.

The calculated function value may be the same value irrespective of the preset wavelength according to the mathematical relationship, or a value that changes according to the preset wavelength. When the calculated function value is a constant, the contribution extent of the excitation light for the corresponding preset wavelength of the measured light profile may be calculated by applying the constant function value to information on the light quantity for each preset wavelength of the reference light profile.

When the calculated function value is a value that changes according to the preset wavelength, the contribution extent of the excitation light for the corresponding preset wavelength of the measured light profile may be calculated by calculating a function value for each preset wavelength and then applying the unique function value to information on the light quantity for each preset wavelength of the reference light profile.

According to an embodiment of the present disclosure, applying the function value to information on the light quantity for each preset wavelength of the reference light profile may be a proportional calculation including multiplying or dividing the light quantity for each preset wavelength by the function value.

### (e) obtaining an emission light profile from (i) the contribution extent determined and (ii) the measured light profile

In the step (e), the emission light profile is provided using the contribution extent of the excitation light profile provided. The determined contribution extent is the contribution extent of an excitation light profile in the measured light profile.

Specifically, the emission light profile is provided by subtracting a light quantity for each preset wavelength of the contribution extent of the excitation light profile from a light quantity for each preset wavelength of the measured light profile. The subtraction is performed for the same preset wavelength. A light quantity for a preset wavelength, in which a light quantity value is only present for the excitation light profile, is not subtracted in any preset wavelength of the measured light profile. In the light quantity for a preset wavelength, in which a light quantity value is only present for the measured light profile, a light quantity value for any preset wavelength of the contribution extent of the excitation light profile is not subtracted.

According to an embodiment of the present disclosure, the step of obtaining the emission light profile may include: (e1) for each preset wavelength of the set of preset wavelengths, subtracting the light quantity of the contribution extent determined from the light quantity of the measured light profile; and (e2) determining the light quantity of the emission light profile for each preset wavelength of the set of preset wavelengths from a result of the step (e1).

In the step (e1), the light quantity of each preset wavelength of the contribution extent of the excitation light profile in the measured light profile is subtracted from the light quantity of corresponding preset wavelength of the measured light profile.

A step of adjusting the scale of information on a light quantity for each preset wavelength may be added before or after the subtraction. The step of adjusting the scale of information on the light quantity may further include, for example, a step of, when information on light quantities for the preset wavelengths are obtained by applying different gains thereto, adjusting the gains.

The step (e2) determines the light quantity of each preset wavelength of the emission light profile from the result of the step (e1).

The light quantity of each preset wavelength of the emission light profile may be determined by forming a set of preset wavelengths including preset wavelengths each including information on the subtracted light quantity obtained in the step (e1). According to an embodiment of the present disclosure, the emission light profile may include information on a light quantity for at least one preset wavelength from among the preset wavelengths including information on the subtracted light quantity and the preset wavelengths which are only present in the measured light profile while being absent in the contribution extent of the excitation light profile.

The emission light profile provided in the step (e) is a profile obtained by removing the contribution extent of the excitation light profile from the measured light profile. The emission light profile obtained in the above step is a profile from which a signal induced by the excitation light generated by the light source unit is removed and only information on a light quantity for a signal induced by the emission light generated by the optical label in the sample positioned in the sample receiving unit remains. The target analyte in the sample is detected using the emission light profile.

### (f) detecting a target analyte in the sample from the emission light profile

The detection of the target analyte is qualitatively or quantitatively detecting or analyzing the target analyte in the sample using the emission light profile provided from the measured light profile obtained for the target analyte by the method according to the present disclosure. The qualitative or quantitative detection may detect or analyze, for example, the presence or absence of the target analyte, the content of the target analyte, or a change in the content or status of the target analyte by biological or chemical reaction.

The detection or analysis of the target analyte and the qualitative or quantitative detection or analysis of the target analyte may refer to obtaining information on the presence or absence of the target analyte, the content of the target analyte, or a change in the content or status of the target analyte by biological or chemical reaction, and may be used interchangeably.

The detection may be performed using the emission light profile itself. Alternatively, the detection may be performed by processing or modifying the emission light profile.

According to an embodiment of the present disclosure, the method according to the present disclosure may repeat the steps (a) to (e) according to the present disclosure for the sample twice or more at different times in accordance with predetermined rules. The target analyte in the sample may be detected by combining a plurality of obtained emission light profiles for the respective repeated operations.

For example, the detection may be performed by a method of determining that a target analyte corresponding to the emission light profile is present in the sample when the level of the emission light profile is equal to or higher than a preset threshold. Alternatively, the detection may be performed by a method of calculating a change in the level of the emission light profile that increases whenever the reaction is repeated and determining whether or not the target analyte is present in accordance with the change calculated. Alternatively, the detection may be performed by a method of (i) obtaining a standard curve for the level of the emission light profile using reference samples in which target analytes of various known concentrations are contained, (ii) comparing the level of the emission light profile provided by the method according to the present disclosure using samples, and (iii) calculating an initial amount of the target analyte contained in each sample.

### II. Optical Spectroscopy-Based Device for Detecting Target Analyte in Sample

According to another aspect of the present disclosure, as such not covered by the claims, provided is an optical spectroscopy-based device for detecting a target analyte in a sample, the device including a light source unit, one or more optical spectrometer units, a sample receiving unit, a first optical path unit, and a second optical path unit. The device for detecting a target analyte in a sample is a device configured to detect a signal from a sample. According to an embodiment of the present disclosure, the signal may be a signal indicating the presence of a target analyte, particularly, a target nucleic acid, in the sample. Thus, the device according to the present disclosure may be a device for detecting the target nucleic acid.

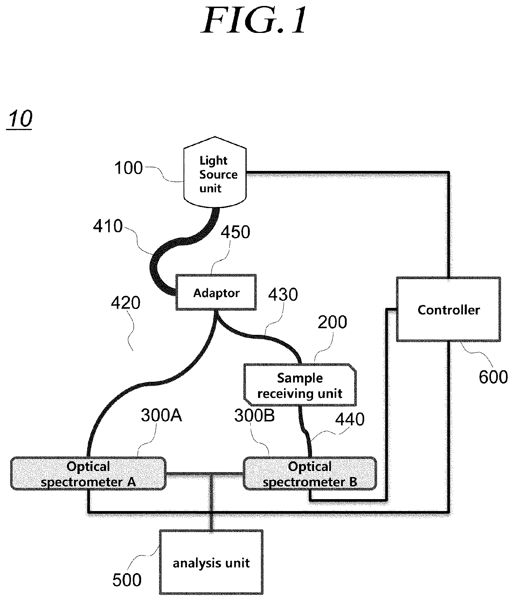

FIG. 1 is a schematic diagram illustrating a device for detecting a target analyte.

Referring to FIG. 1, a detection device 10 according to the present disclosure includes a light source unit 100. The light source unit 100 generate irradiation light to excite an optical label in a sample.

The light source unit 100 provides light energy required for detection. The light source unit 100 includes a light source.

For example, the light source may be a light-emitting diode (LED) unit including an organic LED, an inorganic LED, or a quantum dot LED or a laser unit including a tunable laser, a He-Ne laser, or an Ar laser. The light source may be implemented, for example, as a light source generating monochromatic excitation light or polychromatic excitation light. Regarding the monochromatic excitation, a monochromatic LED (e.g., a blue LED of 470 nm) or a monochromatic laser may be used. Regarding polychromatic excitation, a white LED, a halogen lamp, a xenon lamp, a tungsten-halogen lamp, or a quartz tungsten-halogen lamp may be used. When the light source generating polychromatic excitation light is used, light of an intended excitation wavelength may be provided using a suitable filter. According to an embodiment of the present disclosure, the light source may be an LED.

The light source of the light source unit 100 may be a plurality of light sources. The plurality of light source may be configured to generate light of different wavelengths. In this case, power may be selectively applied to the light sources so that the light source unit 100 may provide light of an intended excitation wavelength. According to an embodiment of the present disclosure, the light source unit 100 may include a plurality of light sources to provide light of different wavelengths.

The detection device 10 according to the present disclosure includes one or more optical spectrometer units 300. Each of the optical spectrometer units 300 is configured to measure the light quantity of each preset wavelength of a set of preset wavelengths.

The optical spectrometer unit 300 may include an optical spectrometer. The optical spectrometer transmits a light quantity signal for each preset wavelength for incident light. According to another embodiment of the present disclosure, the optical spectrometer unit 300 may include one or more detectors. Each of the one or more detectors may be a charge coupled device (CCD), a complementary metal oxide semiconductor field effect transistor (CMOSFET), a photodiode, or the like. The one or more detectors may be configured to detect light of different preset wavelengths.

The optical spectrometer unit 300 may be a plurality of optical spectrometer units 300. As illustrated in FIG. 1, a first optical spectrometer unit 300A may be connected to a first optical path to obtain a reference light profile and a second optical spectrometer unit 300B may be connected to a second optical path to obtain a measured light profile. For example, in a situation in which the detection device 10 includes two sample receiving units 200, the detection device 10 may include three optical spectrometer units 300.

The detection device 10 according to the present disclosure includes a sample receiving unit 200.

The sample receiving unit 200 receives a sample. The sample may be directly contained in the sample receiving unit 200. Alternatively, a reaction vessel, such as a cartridge, a cuvette, or a tube, containing the sample may be received in the sample receiving unit 200. The sample receiving unit 200 includes a sample site. The sample site is a space in which the sample is positioned when the sample or the reaction vessel is received in the sample receiving unit 200.

The sample receiving unit 200 may be configured such that, when the sample or the reaction vessel is received, heat is transferred from the sample receiving unit 200 to the sample or the reaction vessel. For example, the sample receiving unit 200 may include a conductive metal, such as Al, Au, Ag, Ni, or Cu. Alternatively, another configuration than the sample receiving unit 200 may be provided to adjust the temperature of the sample by directly supplying energy to the sample or the reaction vessel. In this case, the sample receiving unit 200 may be configured to receive the sample or the reaction vessel while not transferring heat to the sample or the reaction vessel.

An example of the sample receiving unit 200 is a heat block. The heat block may include a plurality of holes, and reaction vessels may be positioned in the holes, respectively.

Another example of the sample receiving unit 200 is a heating plate. The heating plate is configured such that a thin metal film is contacted to a plate receiving the sample. The heating plate may be operated so that the plate is heated by applying a current to the thin metal film.

Another example of the sample receiving unit 200 is a holder configured to receive one or more chips or a cartridge. An example of the cartridge is a flow cartridge including a flow channel.

The detection device 10 according to the present disclosure includes a first optical path unit and a second optical path unit.

The first optical path unit 410 and 420 forms the first optical path optically connecting the light source unit 100 and the optical spectrometer unit 300. The reference light profile is obtained through the first optical path.

The second optical path unit 410, 430, and 440 forms the second optical path sequentially and optically connecting the light source unit 100, the sample received in the sample receiving unit 200, and the optical spectrometer unit 300. The measured light profile is obtained through the second optical path.

Each of the first optical path and the second optical path may be implemented using a lens and a beam splitter in a conventional manner. In this case, the optical path unit may be configured to include a lens and a beam splitter. According to an embodiment of the present disclosure, the optical path unit may include an optic transmitter, such as an optic cable or an optic fiber.

The first optical path unit and the second optical path unit may share some components. For example, the detection device 10 illustrated in FIG. 1 includes the first optical path unit including the first optic transmitter 410 and the second optic transmitter 420 and the second optical path unit including the first optic transmitter 410, the third optic transmitter 430, and the fourth optic transmitter 440. The first optical path unit and the second optical path unit of the detection device 10 according to an embodiment illustrated in FIG. 1 share the first optic transmitter 410.

For the sharing, the detection device 10 according to the present disclosure may include an adaptor 450. The detection device 1C may include the adaptor 450. The adaptor 450 contacts the ends of two or more optic transmitters, such as optic fibers, thereby allowing light to pass through the two or more optic transmitters. The adaptor 450 is configured to contact one end of the first optic transmitter 410 to one end of each of the second optic transmitter 420 and the third optic transmitter 430, thereby distributing light from the first optic transmitter 410 to the second optic transmitter 420 and the third optic transmitter 430.

The first optic transmitter 410 may be configured such that one end is optically connected to the light source unit 100 and the other end is optically connected to both the second optic transmitter 420 and the third optic transmitter 430. The optical connection between the optic transmitters may be realized using the above-described adaptor 450.

In addition, the other end of the second optic transmitter 420 not connected to the first optic transmitter 410 may be optically connected to the optical spectrometer unit 300A. Thus, the first optical path is formed, and the optical spectrometer unit 300A provides the reference light profile by measuring light from the light source unit 100 for each preset wavelength.

The other end of the third optic transmitter 430 not connected to the first optic transmitter 410 may be optically connected to the sample receiving unit 200. One end of the fourth optic transmitter 440 may be optically connected to the third optic transmitter 430, and the other end of the fourth optic transmitter 440 may be optically connected to the optical spectrometer unit 300B. Thus, the second optical path sequentially and optically connecting the light source unit, the sample received in the sample receiving unit, and the optical spectrometer unit is formed, and the optical spectrometer unit 300B provided the measured light profile by measuring light from the sample received in the sample receiving unit 200.

Reference light and measured light transferred through the two optical paths may be measured by an identical optical spectrometer unit 300. The optical spectrometer unit may be a plurality of optical spectrometer units, and the first optical path and the second optical path may be connected to different optical spectrometer units 300A and 300B, respectively. The embodiment of FIG. 1 illustrates the first optical path and the second optical path connected to different optical spectrometer units 300A and 300B, respectively.

The detection device 10 may be configured such that the first optical path and the second optical path are simultaneously supplied with light from the same light source unit(s) 100. Thus, the reference light profile and the measured light profile may be simultaneously obtained using the same light source unit(s). When the reference light profile and the measured light profile are simultaneously obtained using the same light source unit(s), more accurate measurement is possible. The first optical path and the second optical path may share the first optic transmitter 410 optically connected to the light source unit 100 so as to be simultaneously supplied with light from the same light source unit(s) 100.

According to an embodiment of the present disclosure, the detection device 10 may further include an analysis unit 500 configured to collect and analyze a signal for each preset wavelength of a set of preset wavelengths detected by the one or more optical spectrometer units. The analysis unit 500 collects a reference light profile and a measured light profile obtained by the optical spectrometer units 300A and 300B, analyzes the reference light profile and the measured light profile by the method according to the present disclosure, provides an emission light profile generated from the sample, and detects a target analyte in the sample.

According to an embodiment of the present disclosure, the analysis unit 500 is configured to perform a method of detecting a target analyte in a sample.

The method includes the following steps of: (a) obtaining a reference light profile and a measured light profile, wherein the reference light profile includes information on the light quantity of the light generated by the light source unit for each preset wavelength of a set of preset wavelengths, and the measured light profile includes information on the light quantity of the light measured from the sample for each preset wavelength of the set of preset wavelengths; (b) determining a mathematical relationship between a light quantity for a reference preset wavelength of the reference light profile and a light quantity for the reference preset wavelength of the measured light profile, wherein the reference preset wavelength is a wavelength not including a wavelength of emission light; (c) determining a contribution extent of an excitation light profile in the measured light profile from the reference light profile and the mathematical relationship determined; (d) obtaining an emission light profile from (i) the contribution extent determined and (ii) the measured light profile; and (e) detecting a target analyte in the sample from the emission light profile.

According to an embodiment of the present disclosure, in the detection device 10, a filter may not be disposed in the optical path between the optical spectrometer unit 300 and the sample receiving unit 200. The filter may be a filter preventing specific-wavelength light, of light transmitted from the sample receiving unit 200 to the optical spectrometer unit 300, from being transferred to the optical spectrometer unit 300. Since the detection device 10 according to the present disclosure can effectively provide the emission light profile from the measured light by the method according to the present disclosure, it is possible to detect the target analyte without using a filter for optically separating the emission light.

According to an embodiment of the present disclosure, the detection device 10 may further include a controller 600. The controller 600 may be electrically connected to the light source unit 100 and the optical spectrometer unit 300. The controller 600 adjusts the operation of the light source unit 100 and the operation of the optical spectrometer unit 300 independently of each other. According to an embodiment of the present disclosure, the controller 600 may be configured to control the operation of the device to measure the reference light profile through the first optical path when measuring the measured light profile through the second optical path.

### III. Storage Medium, Device, and Computer Program

According to another aspect of the present disclosure, provided is a computer readable storage medium containing instructions to configure a processor to perform a method of analyzing a target analyte in a sample, the method including the following steps of:
(a) receiving a reference light profile and a measured light profile;
   wherein the reference light profile and the measured light profile are obtained by irradiating a sample with light by generating light by a light source unit of a device for detecting a target analyte, wherein the device for detecting a target analyte includes the light source unit, an optical spectrometer unit, and a sample receiving unit;
   wherein the reference light profile includes information on the light quantity of the light generated by the light source unit for each preset wavelength of a set of preset wavelengths, and the measured light profile includes information on the light quantity of the light measured from the sample for each preset wavelength of the set of preset wavelengths;
(b) determining a mathematical relationship from a light quantity for a reference preset wavelength of the reference light profile and a light quantity for the reference preset wavelength of the measured light profile, wherein the reference preset wavelength is a wavelength not including a wavelength of emission light;
(c) determining a contribution extent of an excitation light profile in the measured light profile from the reference light profile and the mathematical relationship determined;
(d) obtaining an emission light profile from (i) the contribution extent determined and (ii) the measured light profile; and
(e) detecting a target analyte in the sample from the emission light profile.

According to another aspect of the present disclosure, provided is a computer program to be stored on a computer readable storage medium to configure a processor to perform a method of analyzing a target analyte in a sample, the method including the following steps of:
(a) receiving a reference light profile and a measured light profile;
   wherein the reference light profile and the measured light profile are obtained by irradiating a sample with light by generating light by a light source unit of a device for detecting a target analyte, wherein the device for detecting a target analyte includes the light source unit, an optical spectrometer unit, and a sample receiving unit;
   wherein the reference light profile includes information on the light quantity of the light generated by the light source unit for each preset wavelength of a set of preset wavelengths, and the measured light profile includes information on the light quantity of the light measured from the sample for each preset wavelength of the set of preset wavelengths;
(b) determining a mathematical relationship from a light quantity for a reference preset wavelength of the reference light profile and a light quantity for the reference preset wavelength of the measured light profile, wherein the reference preset wavelength is a wavelength not including a wavelength of emission light;
(c) determining a contribution extent of an excitation light profile in the measured light profile from the reference light profile and the mathematical relationship determined;
(d) obtaining an emission light profile from (i) the contribution extent determined and (ii) the measured light profile; and
(e) detecting a target analyte in the sample from the emission light profile.

When the program instructions are executed by the processor, the program instructions allow the processor to perform the above-described method according to the present disclosure. The program instructions for the method of analyzing a target analyte in a sample may include: an instruction to receive the reference light profile and the measured light profile; an instruction to determine the mathematical relationship from the light quantity of the reference preset wavelength of the reference light profile and the light quantity of the reference preset wavelength of the measured light profile; an instruction to determine the contribution extent of the excitation light profile in the measured light profile from the reference light profile and the mathematical relationship determined; and an instruction to provide the emission light profile from (i) the contribution extent determined and (ii) the measured light profile.

The above-described method according to the present disclosure may be built in the processor, e.g., a processor disposed in a standalone computer, a network attached computer, or a data collection device, such as a real-time PCR device.

Examples of the computer readable storage medium include, but are not limited to, various storage media known in the art, e.g., compact disc-recordable (CD-R), a compact disc read-only memory (CD-ROM), a digital versatile disc (DVD), a flash memory, a floppy disk, a hard drive disk (HDD), a portable HDD, a universal series bus (USB) memory, magnetic tape, MiniDisc (MD), a nonvolatile memory card, an EEPROM, an optical disk, an optical storage medium, a random-access memory (RAM), a read-only memory (ROM), a system memory, and a web server.

Datasets used for or produced by performing a method of detecting a target analyte in a sample may be received through various mechanisms. For example, the datasets may be collected by a processor provided in a PCR data collection device. The datasets may be provided to the processor in real time, or may be stored in a memory unit or a buffer to be provided to the processor after an experiment has been completed. Similarly, the datasets may be provided to a separate system, such as a desktop computer system, via a network connection (e.g., a local area network (LAN), a virtual private network (VPN), an intranet, and the Internet) or a direct connection (e.g., a USB memory or another direct wired or wireless connection) to the collection device, or provided on a portable medium, such as a CD, a DVD, a floppy disk, or a portable HDD. Similarly, the datasets may be provided to a server system via a network connection (e.g., an LAN, a VPN, an intranet, the Internet, and a wireless communication network) to a client, such as a notebook or desktop computer system.

The instructions to configure the processor to perform the present disclosure may be included in a logic system. The instructions may be downloaded and stored in a memory module (e.g., an HDD or another memory, such as a local or attached RAM or ROM), although the instructions may be provided on any software storage medium, such as a portable HDD, a USB memory, a floppy disk, a CD, or a DVD. Computers code for implementing the present disclosure may be implemented in a variety of coding languages, such as C, C++, Java, Visual Basic, VBScript, JavaScript, Perl, and XML. In addition, a variety of languages and protocols may be used in external and internal storage and transmission of data and commands according to the present disclosure.

According to another aspect of the present disclosure, provided is a device for analyzing a target analyte in a sample, the device including: (a) a computer processor; and (b) the computer readable storage medium according to the present disclosure coupled to the computer processor.

According to an embodiment of the present disclosure, the device according to the present disclosure may further include a light source unit, an optical spectrometer unit, and a sample receiving unit configured to receive a sample.

According to an embodiment of the present disclosure, the computer processor may receive a reference light profile and a measured light profile, determine a mathematical relationship from the light quantity of a reference preset wavelength of the reference light profile and the light quantity of the reference preset wavelength of the measured light profile, determine a contribution extent of an excitation light profile in the measured light profile from the reference light profile and the mathematical relationship determined, and provide an emission light profile from the contribution extent of the excitation light profile and the measured light profile, so that a target analyte in a sample may be detected. The computer processor may be prepared in such a manner that a single processor can execute all of the above-described performances. Alternatively, the processor unit may be prepared in such a manner that a plurality of processors execute the performances, respectively.

According to an embodiment of the present disclosure, the processor may be embodied by installing software into a conventional device (e.g., a real-time PCR device) used for detecting a target analyte (e.g., a target nucleic acid molecule).

According to an embodiment of the present disclosure, the target analyte in the sample is detected by obtaining the reference light profile and the measured light profile, determining the mathematical relationship from the light quantities of the reference preset wavelengths of the light profiles, and obtaining the emission light profile from the measured light profile by calculating the contribution extent of the excitation light profile in the measured light profile using the mathematical relationship.

### Example

In Example of the present disclosure, it was examined whether or not an emission light profile included in a measured light profile was derived by the method according to the present disclosure by obtaining a reference light profile and the measured light profile using an optical label.

### Obtaining reference light profile and measured light profile

The target analyte used in Example was a probe with FAM molecules bound thereto. A detection module of a detection device used for the measurement was prepared as illustrated in FIG. 1. The light source unit was implemented as an LED to generate irradiation light of a wavelength ranging from about 400 nm to about 590 nm. The optical spectrometer unit may measure the light quantities of light detected through three channels by dividing the light detected into a total of 18 preset wavelengths. The preset wavelengths measured are as illustrated in FIGS. 2 and 3. Two same optical spectrometer units were used, and the reference light profile and the measured light profile were obtained from the optical spectrometer units, respectively.

A sample was positioned in a sample receiving unit, and light was generated by applying power to a light source. Light quantities for respective preset wavelengths were measured by an optical spectrometer unit A measuring light that has not passed through the sample receiving unit and an optical spectrometer unit B measuring light that has passed through the sample receiving unit.

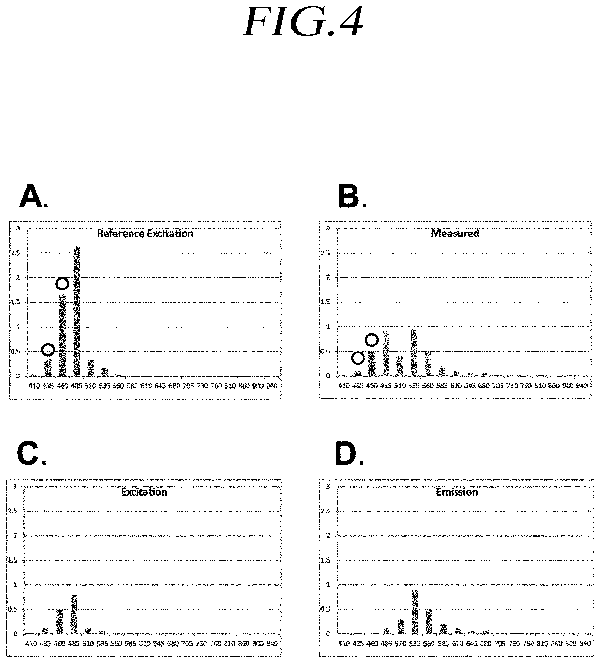

FIG. 4A is a graph illustrating light quantities of the reference light profile obtained by the optical spectrometer unit A for respective preset wavelengths. FIG. 4B is a graph illustrating light quantities of the measured light profile obtained by the optical spectrometer unit B for respective preset wavelengths. As can be seen in FIG. 3, the light quantities of the reference light were measured for a total of 7 preset wavelengths, i.e., from a first preset wavelength to a seventh preset wavelength, and the light quantities of the measured light were measured for a total of 11 preset wavelengths, i.e., from a first preset wavelength to an eleventh preset wavelength.

### Determining mathematical relationship

Wavelengths of light emitted by commercially available optical labels are commonly known. The wavelengths of emission light of the FAM range from about 460 nm to about 700 nm. Thus, the reference preset wavelengths were determined to be the second preset wavelength and the third preset wavelength respectively not including any wavelength of the emission light.

Afterwards, a relationship was calculated by comparing the light quantities of the reference light profile and the measured light profile for the second preset wavelength and the third preset wavelength. In FIGS. 4A and 4B, the light quantities of the reference preset wavelengths of the reference light profile and the measured light profile were indicated by open circles.

As a result of the calculation, it was confirmed that the light quantities of the reference light and the measured light for the second preset wavelength are in a 3:1 ratio relationship, and the light quantities of the reference light and the measured light for the third preset wavelength are also in a 3:1 ratio relationship. Thus, it was confirmed that the mathematical relationship for the sample and the optical label used in the example of the present disclosure is not influenced by the preset wavelengths designated as the reference preset wavelength. Accordingly, the mathematical relationship was determined to be [light quantity of excitation light in measured light = light quantity of reference light profile X 3], with a coefficient for preset wavelengths being zero (0).

### Providing contribution extent of excitation light profile

The contribution extent of the excitation light profile in the measured light profile was provided from the reference light profile and the mathematical relationship determined. The contribution extent of the excitation light profile is provided in a form including information on a light quantity for each preset wavelength of a set of preset wavelengths.

The contribution extent of the excitation light profile was obtained by obtaining converted light quantities for respective preset wavelengths by applying the light quantities of the reference light profile for respective preset wavelengths to the mathematical relationship determined.

As a result, the contribution extent of the excitation light profile included in the measured light profile illustrated in FIG. 4B was calculated as in FIG. 4C.

### Providing the emission light profile

An emission light profile was calculated from the measured light profile using the contribution extent of the excitation light profile provided.

The calculation was performed by a method of subtracting the contribution extent of the excitation light profile illustrated in FIG. 4C from the emission light profile illustrated in FIG. 4B.

As a result, the emission light profile as illustrated in FIG. 4D was calculated.

Also provided is a device, as such not covered by the claims, for detecting a target analyte in a sample using spectrometry, the device including: a light source unit; one or more optical spectrometer units; a sample receiving unit configured to receive a sample; a first optical path unit defining a first optical path optically connecting the light source unit and the optical spectrometer units; and a second optical path unit defining a second optical path sequentially and optically connecting the light source unit, the sample received in the sample receiving unit, and the optical spectrometer unit, wherein the optical spectrometer units may measure a light quantity for each preset wavelength of a set of preset wavelengths, a reference light profile may be obtained through the first optical path, a measured light profile may be obtained through the second optical path, the reference light profile includes information on the light quantity of the light generated by the light source unit for each preset wavelength of the set of preset wavelengths, and the measured light profile includes information on the light quantity of the light measured from the sample for each preset wavelength of the set of preset wavelengths.

Although the present disclosure has been described in detail with reference to the specific features, it would be obvious to those having ordinary knowledge in the art that this description is only illustrative and the scope of the present disclosure is not limited thereto. Therefore, the substantial scope of the present disclosure will be defined by the appended claims and their equivalents.

### [CROSS-REFERENCE TO RELATED APPLICATION(S)]

This application claims priority to Korean Patent Application No. 10-2020-0188382, filed on December 30, 2020, in the Korean Intellectual Property Office.

## Claims

1. A method of detecting a target analyte in a sample, the method comprising:
(a) irradiating a sample with light by generating light by a light source unit **(100)** of a device **(10)** for detecting a target analyte, wherein the device for detecting a target analyte comprises the light source unit, an optical spectrometer unit **(300),** and a sample receiving unit **(200);**
(b) obtaining a reference light profile and a measured light profile, wherein the reference light profile comprises information on light quantity of the light generated by the light source unit for each preset wavelength of a set of preset wavelengths, and the measured light profile comprises information on light quantity of the light measured from the sample for each preset wavelength of the set of preset wavelengths,
**characterized in that the method further comprises:**
(c) determining a mathematical relationship between light quantity for a reference preset wavelength of the reference light profile and light quantity for the reference preset wavelength of the measured light profile, wherein the reference preset wavelength is a wavelength not including a wavelength of emission light;
(d) determining a contribution extent of an excitation light profile in the measured light profile from the reference light profile and the mathematical relationship determined;
(e) obtaining an emission light profile from (i) the contribution extent determined and (ii) the measured light profile; and
(f) detecting a target analyte in the sample from the emission light profile.

2. The method of claim 1, wherein the step (e) of obtaining the emission light profile comprises:
(e1) for each preset wavelength of the set of preset wavelengths, subtracting light quantity of the contribution extent determined from light quantity of the measured light profile; and
(e2) determining light quantity of the emission light profile for each preset wavelength of the set of preset wavelengths from a result of the step (e1).

3. The method of claim 1, wherein the reference light profile and the measured light profile are obtained using the same light source unit.

4. The method of claim 1, wherein the reference light profile and the measured light profile are simultaneously obtained using the same light source unit.

5. The method of claim 1, wherein the reference light profile and the measured light profile are obtained using different optical spectrometer units.

6. The method of claim 1, wherein obtaining the reference light profile and the measured light profile comprises obtaining a plurality of reference light profiles and a plurality of measured light profiles in different measurement conditions.

7. A computer readable storage medium containing instructions to configure a processor to perform a method of analyzing a target analyte in a sample, the method comprising:
(a) receiving a reference light profile and a measured light profile;
wherein the reference light profile and the measured light profile are obtained by irradiating a sample with light by generating light by a light source unit **(100)** of a device **(10)** for detecting a target analyte, wherein the device for detecting a target analyte comprises the light source unit, an optical spectrometer unit **(300),** and a sample receiving unit **(200)**;
wherein the reference light profile comprises information on light quantity of the light generated by the light source unit for each preset wavelength of a set of preset wavelengths, and the measured light profile comprises information on light quantity of the light measured from the sample for each preset wavelength of the set of preset wavelengths,
**characterized in that the method further comprises:**
(b) determining a mathematical relationship from light quantity for a reference preset wavelength of the reference light profile and light quantity for the reference preset wavelength of the measured light profile, wherein the reference preset wavelength is a wavelength not including a wavelength of emission light;
(c) determining a contribution extent of an excitation light profile in the measured light profile from the reference light profile and the mathematical relationship determined;
(d) obtaining an emission light profile from (i) the contribution extent determined and (ii) the measured light profile; and
(e) detecting a target analyte in the sample from the emission light profile.

8. A device for analyzing a target analyte in a sample, the device comprising:
(a) a computer processor; and
(b) the computer readable storage medium of claim 7 coupled to the computer processor.

9. A computer program to be stored on a computer readable storage medium to configure a processor to perform a method of analyzing a target analyte in a sample, the method comprising:
(a) receiving a reference light profile and a measured light profile;
wherein the reference light profile and the measured light profile are obtained by irradiating a sample with light by generating light by a light source unit **(100)** of a device **(10)** for detecting a target analyte, wherein the device for detecting a target analyte comprises the light source unit, an optical spectrometer unit **(300),** and a sample receiving unit **(200);**
wherein the reference light profile comprises information on light quantity of the light generated by the light source unit for each preset wavelength of a set of preset wavelengths, and the measured light profile comprises information on light quantity of the light measured from the sample for each preset wavelength of the set of preset wavelengths,
**characterized in that the method further comprises:**
(b) determining a mathematical relationship from light quantity for a reference preset wavelength of the reference light profile and light quantity for the reference preset wavelength of the measured light profile, wherein the reference preset wavelength is a wavelength not including a wavelength of emission light;
(c) determining a contribution extent of an excitation light profile in the measured light profile from the reference light profile and the mathematical relationship determined;
(d) obtaining an emission light profile from (i) the contribution extent determined and (ii) the measured light profile; and
(e) detecting a target analyte in the sample from the emission light profile.

## Patentansprüche

1. Verfahren zum Nachweis eines Zielanalyten in einer Probe, wobei das Verfahren umfasst:
(a) Bestrahlen einer Probe mit Licht durch Erzeugen von Licht durch eine Lichtquelleneinheit **(100)** einer Vorrichtung **(10)** zum Erkennen eines Zielanalyten, wobei die Vorrichtung zum Erkennen eines Zielanalyten die Lichtquelleneinheit, eine optische Spektrometereinheit **(300)** und eine Probenaufnahmeeinheit **(200)** umfasst;
(b) Erhalten eines Referenzlichtprofils und eines gemessenen Lichtprofils, wobei das Referenzlichtprofil Informationen über die Lichtmenge des von der Lichtquelleneinheit für jede voreingestellte Wellenlänge eines Satzes voreingestellter Wellenlängen erzeugten Lichts umfasst, und das gemessene Lichtprofil Informationen über die Lichtmenge des aus der Probe gemessenen Lichts für jede voreingestellte Wellenlänge des Satzes voreingestellter Wellenlängen umfasst,
**dadurch gekennzeichnet, dass das Verfahren ferner umfasst:**
(c) Bestimmen einer mathematischen Beziehung zwischen einer Lichtmenge für eine voreingestellte Referenzwellenlänge des Referenzlichtprofils und einer Lichtmenge für die voreingestellte Referenzwellenlänge des gemessenen Lichtprofils, wobei die voreingestellte Referenzwellenlänge eine Wellenlänge ist, die keine Wellenlänge von Emissionslicht enthält;
(d) Bestimmen eines Beitragsumfangs eines Anregungslichtprofils in das gemessene Lichtprofil aus dem Referenzlichtprofil und der bestimmten mathematischen Beziehung;
(e) Erhalten eines Emissionslichtprofils aus (i) dem bestimmten Beitragsumfang und (ii) dem gemessenen Lichtprofil; und
(f) Erkennen eines Zielanalyten in der Probe aus dem Emissionslichtprofil.

2. Verfahren nach Anspruch 1, wobei der Schritt (e) zum Erhalten des Emissionslichtprofils umfasst:
(e1) für jede voreingestellte Wellenlänge des Satzes voreingestellter Wellenlängen, Subtrahieren der Lichtmenge des von der Lichtmenge des gemessenen Lichtprofils bestimmten Beitragsumfangs; und
(e2) Bestimmen der Lichtmenge des Emissionslichtprofils für jede voreingestellte Wellenlänge des Satzes voreingestellter Wellenlängen aus einem Ergebnis des Schritts (e1).

3. Verfahren nach Anspruch 1, wobei das Referenzlichtprofil und das gemessene Lichtprofil unter Verwendung derselben Lichtquelleneinheit erhalten werden.

4. Verfahren nach Anspruch 1, wobei das Referenzlichtprofil und das gemessene Lichtprofil gleichzeitig unter Verwendung derselben Lichtquelleneinheit erhalten werden.

5. Verfahren nach Anspruch 1, wobei das Referenzlichtprofil und das gemessene Lichtprofil unter Verwendung verschiedener optischer Spektrometereinheiten erhalten werden.

6. Verfahren nach Anspruch 1, wobei das Erhalten des Referenzlichtprofils und des gemessenen Lichtprofils das Erhalten einer Vielzahl von Referenzlichtprofilen und einer Vielzahl von gemessenen Lichtprofilen unter verschiedenen Messbedingungen umfasst.

7. Computerlesbares Speichermedium mit Anweisungen zum Konfigurieren eines Prozessors zum Durchführen eines Verfahrens zum Analysieren eines Zielanalyten in einer Probe, wobei das Verfahren umfasst:
(a) Empfangen eines Referenzlichtprofils und eines gemessenen Lichtprofils;
wobei das Referenzlichtprofil und das gemessene Lichtprofil durch Bestrahlen einer Probe mit Licht durch Erzeugen von Licht durch eine Lichtquelleneinheit **(100)** einer Vorrichtung **(10)** zum Erkennen eines Zielanalyten erhalten werden, wobei die Vorrichtung zum Erkennen eines Zielanalyten die Lichtquelleneinheit, eine optische Spektrometereinheit **(300)** und eine Probenaufnahmeeinheit **(200)** umfasst;
wobei das Referenzlichtprofil Informationen über die Lichtmenge des von der Lichtquelleneinheit für jede voreingestellte Wellenlänge eines Satzes voreingestellter Wellenlängen erzeugten Lichts umfasst, und das gemessene Lichtprofil Informationen über die Lichtmenge des aus der Probe gemessenen Lichts für jede voreingestellte Wellenlänge des Satzes voreingestellter Wellenlängen umfasst,
**dadurch gekennzeichnet, dass das Verfahren ferner umfasst:**
(b) Bestimmen einer mathematischen Beziehung aus einer Lichtmenge für eine voreingestellte Referenzwellenlänge des Referenzlichtprofils und einer Lichtmenge für die voreingestellte Referenzwellenlänge des gemessenen Lichtprofils, wobei die voreingestellte Referenzwellenlänge eine Wellenlänge ist, die keine Wellenlänge von Emissionslicht enthält;
(c) Bestimmen eines Beitragsumfangs eines Anregungslichtprofils in das gemessene Lichtprofil aus dem Referenzlichtprofil und der bestimmten mathematischen Beziehung;
(d) Erhalten eines Emissionslichtprofils aus (i) dem bestimmten Beitragsumfang und (ii) dem gemessenen Lichtprofil; und
(e) Erkennen eines Zielanalyten in der Probe aus dem Emissionslichtprofil.

8. Vorrichtung zum Analysieren eines Zielanalyten in einer Probe, wobei die Vorrichtung umfasst:
(a) einen Computerprozessor; und
(b) das computerlesbares Speichermedium nach Anspruch 7, das mit dem Computerprozessor gekoppelt ist.

9. Computerprogramm, das auf einem computerlesbaren Speichermedium gespeichert werden soll, um einen Prozessor so zu konfigurieren, dass er ein Verfahren zum Analysieren eines Zielanalyten in einer Probe durchführt, wobei das Verfahren umfasst:
(a) Empfangen eines Referenzlichtprofils und eines gemessenen Lichtprofils;
wobei das Referenzlichtprofil und das gemessene Lichtprofil durch Bestrahlen einer Probe mit Licht durch Erzeugen von Licht durch eine Lichtquelleneinheit **(100)** einer Vorrichtung **(10)** zum Erkennen eines Zielanalyten erhalten werden, wobei die Vorrichtung zum Erkennen eines Zielanalyten die Lichtquelleneinheit, eine optische Spektrometereinheit **(300)** und eine Probenaufnahmeeinheit **(200)** umfasst;
wobei das Referenzlichtprofil Informationen über die Lichtmenge des von der Lichtquelleneinheit für jede voreingestellte Wellenlänge eines Satzes voreingestellter Wellenlängen erzeugten Lichts umfasst, und das gemessene Lichtprofil Informationen über die Lichtmenge des aus der Probe gemessenen Lichts für jede voreingestellte Wellenlänge des Satzes voreingestellter Wellenlängen umfasst,
**dadurch gekennzeichnet, dass das Verfahren ferner umfasst:**
(b) Bestimmen einer mathematischen Beziehung aus einer Lichtmenge für eine voreingestellte Referenzwellenlänge des Referenzlichtprofils und einer Lichtmenge für die voreingestellte Referenzwellenlänge des gemessenen Lichtprofils, wobei die voreingestellte Referenzwellenlänge eine Wellenlänge ist, die keine Wellenlänge von Emissionslicht enthält;
(c) Bestimmen eines Beitragsumfangs eines Anregungslichtprofils in das gemessene Lichtprofil aus dem Referenzlichtprofil und der bestimmten mathematischen Beziehung;
(d) Erhalten eines Emissionslichtprofils aus (i) dem bestimmten Beitragsumfang und (ii) dem gemessenen Lichtprofil; und
(e) Erkennen eines Zielanalyten in der Probe aus dem Emissionslichtprofil.

## Revendications

1. Procédé de détection d'un analyte cible dans un échantillon, le procédé comportant :
(a) l'irradiation d'un échantillon avec de la lumière en générant de la lumière par une unité de source de lumière **(100)** d'un dispositif **(10)** de détection d'un analyte cible, dans lequel le dispositif de détection d'un analyte cible comporte l'unité de source de lumière, une unité de spectromètre optique **(300)** et une unité de réception d'échantillon **(200)** ;
(b) l'obtention d'un profil de lumière de référence et d'un profil de lumière mesuré, dans lequel le profil de lumière de référence comporte des informations sur la quantité de lumière de la lumière générée par l'unité de source de lumière pour chaque longueur d'onde prédéfinie d'un ensemble de longueurs d'onde prédéfinies, et le profil de lumière mesuré comporte des informations sur la quantité de lumière de la lumière mesurée à partir de l'échantillon pour chaque longueur d'onde prédéfinie de l'ensemble de longueurs d'onde prédéfinies,
**caractérisé en ce que le procédé comporte en outre** :
(c) la détermination d'une relation mathématique entre une quantité de lumière pour une longueur d'onde prédéfinie de référence du profil de lumière de référence et une quantité de lumière pour la longueur d'onde prédéfinie de référence du profil de lumière mesuré, la longueur d'onde prédéfinie de référence étant une longueur d'onde n'incluant pas une longueur d'onde de lumière d'émission ;
(d) la détermination d'une étendue de contribution d'un profil de lumière d'excitation dans le profil de lumière mesuré à partir du profil de lumière de référence et de la relation mathématique déterminée ;
(e) l'obtention d'un profil de lumière d'émission à partir de (i) l'étendue de contribution déterminée et (ii) le profil de lumière mesuré ; et
(f) la détection d'un analyte cible dans l'échantillon à partir du profil d'émission de lumière.

2. Procédé selon la revendication 1, dans lequel l'étape (e) d'obtention du profil de lumière d'émission comporte :
(e1) pour chaque longueur d'onde prédéfinie de l'ensemble de longueurs d'onde prédéfinies, la soustraction de la quantité de lumière de l'étendue de contribution déterminée de la quantité de lumière du profil de lumière mesuré ; et
(e2) la détermination de la quantité de lumière du profil de lumière d'émission pour chaque longueur d'onde prédéfinie de l'ensemble de longueurs d'onde prédéfinies à partir d'un résultat de l'étape (e1).

3. Procédé selon la revendication 1, dans lequel le profil de lumière de référence et le profil de lumière mesuré sont obtenus en utilisant la même unité de source de lumière.

4. Procédé selon la revendication 1, dans lequel le profil de lumière de référence et le profil de lumière mesuré sont obtenus simultanément en utilisant la même unité de source de lumière.

5. Procédé selon la revendication 1, dans lequel le profil de lumière de référence et le profil de lumière mesuré sont obtenus à l'aide de différentes unités de spectromètre optique.

6. Procédé selon la revendication 1, dans lequel l'obtention du profil de lumière de référence et du profil de lumière mesuré comporte l'obtention d'une pluralité de profils de lumière de référence et d'une pluralité de profils de lumière mesurés dans différentes conditions de mesure.

7. Support de stockage lisible par ordinateur contenant des instructions pour configurer un processeur afin de réaliser un procédé d'analyse d'un analyte cible dans un échantillon, le procédé comportant :
(a) la réception d'un profil de lumière de référence et d'un profil de lumière mesuré ;
dans lequel le profil de lumière de référence et le profil de lumière mesuré sont obtenus par irradiation d'un échantillon avec de la lumière en générant de la lumière par une unité de source de lumière **(100)** d'un dispositif **(10)** de détection d'un analyte cible, dans lequel le dispositif de détection d'un analyte cible comporte l'unité de source de lumière, une unité de spectromètre optique **(300)** et une unité de réception d'échantillon **(200)** ;
dans lequel le profil de lumière de référence comporte des informations sur la quantité de lumière de la lumière générée par l'unité de source de lumière pour chaque longueur d'onde prédéfinie d'un ensemble de longueurs d'onde prédéfinies, et le profil de lumière mesuré comporte des informations sur la quantité de lumière de la lumière mesurée à partir de l'échantillon pour chaque longueur d'onde prédéfinie de l'ensemble de longueurs d'onde prédéfinies,
**caractérisé en ce que le procédé comporte en outre** :
(b) la détermination d'une relation mathématique à partir d'une quantité de lumière pour une longueur d'onde prédéfinie de référence du profil de lumière de référence et d'une quantité de lumière pour la longueur d'onde prédéfinie de référence du profil de lumière mesuré, la longueur d'onde prédéfinie de référence étant une longueur d'onde n'incluant pas une longueur d'onde de lumière d'émission ;
(c) la détermination d'une étendue de contribution d'un profil de lumière d'excitation dans le profil de lumière mesuré à partir du profil de lumière de référence et de la relation mathématique déterminée ;
(d) l'obtention d'un profil de lumière d'émission à partir de (i) l'étendue de contribution déterminée et (ii) le profil de lumière mesuré ; et
(e) la détection d'un analyte cible dans l'échantillon à partir du profil d'émission de lumière.

8. Dispositif d'analyse d'un analyte cible dans un échantillon, le dispositif comportant :
(a) un processeur informatique ; et
(b) le support de stockage lisible par ordinateur selon la revendication 7 couplé au processeur informatique.

9. Programme informatique à stocker sur un support de stockage lisible par ordinateur pour configurer un processeur afin de réaliser un procédé d'analyse d'un analyte cible dans un échantillon, le procédé comportant :
(a) la réception d'un profil de lumière de référence et d'un profil de lumière mesuré ;
dans lequel le profil de lumière de référence et le profil de lumière mesuré sont obtenus par irradiation d'un échantillon avec de la lumière en générant de la lumière par une unité de source de lumière **(100)** d'un dispositif **(10)** de détection d'un analyte cible, dans lequel le dispositif de détection d'un analyte cible comporte l'unité de source de lumière, une unité de spectromètre optique **(300)** et une unité de réception d'échantillon **(200)** ;
dans lequel le profil de lumière de référence comporte des informations sur la quantité de lumière de la lumière générée par l'unité de source de lumière pour chaque longueur d'onde prédéfinie d'un ensemble de longueurs d'onde prédéfinies, et le profil de lumière mesuré comporte des informations sur la quantité de lumière de la lumière mesurée à partir de l'échantillon pour chaque longueur d'onde prédéfinie de l'ensemble de longueurs d'onde prédéfinies,
**caractérisé en ce que le procédé comporte en outre** :
(b) la détermination d'une relation mathématique à partir d'une quantité de lumière pour une longueur d'onde prédéfinie de référence du profil de lumière de référence et d'une quantité de lumière pour la longueur d'onde prédéfinie de référence du profil de lumière mesuré, la longueur d'onde prédéfinie de référence étant une longueur d'onde n'incluant pas une longueur d'onde de lumière d'émission ;
(c) la détermination d'une étendue de contribution d'un profil de lumière d'excitation dans le profil de lumière mesuré à partir du profil de lumière de référence et de la relation mathématique déterminée ;
(d) l'obtention d'un profil de lumière d'émission à partir de (i) l'étendue de contribution déterminée et (ii) le profil de lumière mesuré ; et
(e) la détection d'un analyte cible dans l'échantillon à partir du profil d'émission de lumière.
